# EUROPEAN PATENT SPECIFICATION

(11) **EP 0 716 807 B1**
(45) Date of publication and mention of the grant of the patent: **11.07.2001**
(21) Application number: 95119715.1
(22) Date of filing: 14.12.1995
(51) Int. Cl.: A01M 1/20, A61L 9/03

(54) **Heater for evaporators of solid or liquid insecticide or deodorant formulations**
Heizelement für Verdampfern von festen oder flüssigen Insektiziden oder Deodorantien
Dispositif de chauffage pour évaporateur d'insecticides ou déodorants solides ou liquides

(30) Priority: 16.12.1994 IT MI942547
(43) Date of publication of application: 19.06.1996
(73) Proprietor: ZOBELE INDUSTRIE CHIMICHE S.p.A., I-38100 Trento (IT)
(72) Inventor: Zobele, Franco, I-38100 Trento (IT)
(74) Representative: Faggioni, Marco, Dr. Ing.

(56) References cited:
- EP-A- 0 290 159
- EP-A- 0 334 785
- EP-A- 0 420 144
- DE-U- 9 102 695
- US-A- 4 663 315
- US-A- 4 758 708
- US-A- 5 222 186

## Description

The present invention concerns a heating device for evaporators of insecticide or deodorant substances both in solid and in liquid formulations. In the first case, the active ingredient is absorbed into solid supports of pressed cardboard, having a characteristic thin rectangular shape, commonly called "mats". In the second case, the active ingredient is in the form of a liquid solution contained into a closed vessel, ending at the top with a cylindrical wick apt to draw therein the liquid solution by capillary effect.

The vaporizable liquid insecticide formulations were introduced on the market subsequently to the mats, to answer the requirement of being able to dispose of a longer Lasting product. In fact, while the mats wear out more or less in one night, the liquid formulations last much longer, for instance from 30 to 60 nights. It is evident that the technical development of these formulations has been simultaneously accompanied by the development of the respective heating devices, commonly and simply called "heaters".

The first ones to be developed were thus the mat heaters, initially consisting of a metal plate having the same dimensions as the insecticide mat, one or more resistors being positioned below said plate to obtain the desired heating effect. Subsequently, to obtain an improved and more homogeneous heat distribution, as well as a steadier housing for the resistors and a higher safety in use, it was proposed to produce heaters of ceramic material, comprising recesses or holes to house the resistors and having an upper flat and continuos surface over which the mat was positioned (IT-B-1050766). More recently, besides using the conventional coil resistors, it has been found appropriate to use posistors (PTC, i.e. Positive Temperature Coefficient heating device), consisting of cylindrical or rectangular pellets of pressed granular conductive material, which allow to obtain an automatic regulation of the heat emitted since their electric resistance decreases as temperature increases.

In the field of evaporators for liquid formulations contained in a vessel, the electric heating device has evidently taken up, since the beginning, quite a different shape. In fact, the problem in this case is to homogeneously heat the liquid solution which rises into the wick by capillarity, without causing any local superheatings of said wick which would lead to a premature carbonization of the active ingredient thereon and thereby inhibit its capability of further absorption. This problem has been solved with different solutions, but two types are fundamentally adopted: heaters with rectilinear resistors (or PTC) positioned laterally to the wick (EP-A-420144); heaters with ring resistors positioned around the wick (US-A-4663315; EP-A-334785).

The best results have obviously been obtained with the heaters of the second type, which allow a perfectly homogeneous heating of the wick; nonetheless, such type of heater has only had a scarce diffusion, and has now been practically abandoned, on account of the high production cost of a ring resistor and, also, of its higher fragility; the market has thus shifted onto heaters with a lateral rectilinear resistor, preferably buried into a ceramic support. Said support is either positioned to the side of the wick, or it partly, or even totally surrounds the wick, so as to compensate for the asymmetrical positioning of the heat source in respect of said wick.

Together with these two types of heaters, described heretofore, the market has started to offer also heaters apt to be used both with liquid formulations and with mats - so-called double-use heaters - which allow to satisfy different temporary requirements of the user, as well as to anyhow use the evaporator, independently from whether either one of the two types of insecticide formulations may or not be locally available. This type of heater is substantially equal to the mat heaters with ceramic support, said support comprising however a through passage to allow housing therein the wick of the liquid insecticide formulations.

The heaters of the aforementioned type are nevertheless not free from drawbacks, all substantially tied to the intrinsic characteristics of workability of the ceramic article. In fact, to start with, the ceramic supports of heaters undergo considerable shrinkage and deformation while baking in the oven, and should thus be dimensioned with fairly wide tolerances. For instance, for pieces having the size of a conventional support for heaters (about 30x40 mm), the position of the hole axis housing the wick - in an ordinary manufacturing process usually carried out for pieces of this type and worth - has a tolerance of ± 1.5 mm. Due to these high tolerance values, it is not possible to guarantee, while using the heater, a constant and accurate centering of the wick into its housing hole formed in the ceramic support, since the clearance existing between the wick and the hole is normally just of 1.5 mm. The consequence of an undesirable adherence between the wick and the hole will thus be both the carbonization of said wick, where it gets in contact with the walls of the support, and the fouling of said walls, leading to an overall reduced efficiency of the heater. In any case, even when there is no direct contact between the wick and the hole, the non perfectly centered positioning of these two elements results into a non-homogeneous heating of the wick and, thus, a less efficient performance of the heater.

Another considerable drawback of ceramic supports lies in the intrinsically fragile nature of this material, which does not allow it to be formed with projecting parts, or with scarcely thick parts, which could instead be useful for an improved functionality of the heater.

The present invention thus relates to the aforementioned type of double-use heaters, and proposes to supply a heater produced both with plastic and with ceramic materials, whose shape and arrangement have been carefully studied in order to obtain the utmost efficiency thereof.

According to the present invention, said object is reached by means of a heater for evaporators of insecticide or deodorant formulations both in solid mat form and in liquid form with a wick - of the type comprising at least one electric heating element, a flat surface for positioning the mat, and a housing for the wick - characterized in that it comprises a ceramic block, into which is inserted and fixed said electric heating element, and a support base of plastic material consisting of a plate, apt to form said flat surface, and of thin walls projecting from said plate, apt to define a frame for housing said wick as well as a cage for containing said ceramic block.

The invention will now be described in further detail, with reference to a preferred embodiment thereof, illustrated by way of example on the accompanying drawings, in which:
Fig. 1 is a plan view of the heater of the invention, seen from one side, with the respective plug for connection to the electric system;
Fig. 2 is a plan view of the other side of the heater shown in fig. 1;
Fig. 3 is a lateral view of the heater, in the direction of the arrow F of fig. 1;
Fig. 4 is a cross-section view of the heater, along the line IV-IV of fig. 1; and
Fig. 5 is a cross-section view of the heater, along the line V-V of fig. 1.

As clearly shown on the drawings, the heater of the present invention substantially comprises a support base 1, made of plastic material and comprising a plate 1a from which project thin walls apt to define a cage 2, into which is inserted and firmly fixed a ceramic block 3 housing an electric heating element (not shown), and a frame 4 to house the wick of a liquid formulation. Preferably, the frame 4 is adjacent to one of the walls of the cage 2 for a length corresponding more or less to the width of the wick and, in correspondence of said length, the frame 4 and said cage wall almost totally interrupt, so that the ceramic block 3 directly faces into the cavity defined by the frame 4.

This innovating solution, studied by the Applicant, allows to overcome the aforementioned drawbacks of the prior art, without substantially reducing the thermal efficiency of the heater in respect of the known type heaters entirely made of ceramic material.

In fact, by a suitable choice of the plastic material it is possible to obtain excellent mechanical characteristics, such as a high dimensional precision of the pieces formed by moulding, determined by extremely low shrinking coefficients and, at the same time, excellent thermal characteristics, such as a strong resistance to high temperatures with no degradation or dimensional variations. The thermal conductivity of the material does not appear to be a critical factor, since all the walls of the support base 1 are scarcely thick and, furthermore, the heating of the formulations, both liquid and solid, is preferably obtained by convection rather than by conduction. A preferred material, having these characteristics, consists for example of a phenilene-polysulphide based plastic material (FPS).

By using materials of the type described heretofore, the support base 1 can be produced with extremely low tolerances, for instance of the order of hundredths of millimeters. This allows to guarantee a high precision in centering the wick in its housing seat, which precision keeps constant for all the pieces being produced. Thanks to the very good moldability of the plastic material, it is moreover possible to form the support base 1 with a series of structural details and of additional parts, which it would have been quite unthinkable to realize in the traditional ceramic heaters and which contribute to notably increase the functionality of the heater of the present invention.

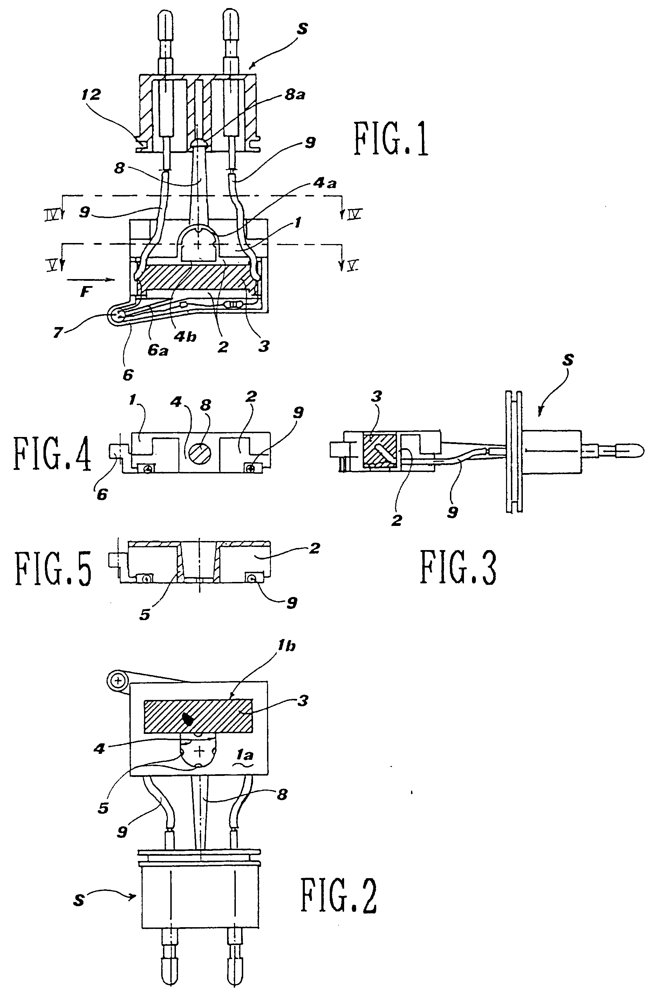

According to the invention, the support base 1 thus comprises a flat plate 1a - for positioning the solid mat formulations - which comprises a large window 1b in correspondence of the ceramic heating block 3. Said block 3 is preferably shaped as a long parallelepipedon with a square section, and the dimensions of the window 1b are slightly smaller than those of the corresponding surface of the block 3, so as to prevent it from sliding out of the window. Thin walls project from the plate 1a, said walls being apt to define, together with the window 1b, a cage 2 to elastically contain the block 3. For this purpose, the walls are provided along three sides of the block 3 and, precisely, one of the short sides and the two Long sides; the walls on the long sides comprise furthermore terminal ribs ending at right angles, as can be seen in fig. 1, to prevent the block 3 from sliding out of the bottom part of the heater. From the above, it is quite evident that the block 3 is inserted into the cage 2 sideways - in the direction of arrow F - in correspondence of the only side of the cage 2 which is not closed by a wall, as can be clearly seen in fig. 3.

When the heater of the present invention is used with solid mat formulations, the heat emitted by the ceramic block 3 comes out of the window 1b with a hot air stream which envelops the mat and heats it by convection. To favour this type of heating, grooves (not shown) can be provided on the plate 1a of the support base 1, such grooves ending into the window 1b and being therefore apt to form preferential channels for the hot air stream produced by the block 3.

Said stream is of course favoured by the fact that the block 3 is in a lower position in respect of the plate 1a onto which bears the mat.

As specified heretofore, also the seat housing the wick is formed into the support base 1. Said seat consists of a frame 4, delimited on three sides by a thin arched wall 4a, projecting from the plate 1a, and on the fourth side by one of the walls of the cage 2, containing the ceramic block 3, and precisely the wall positioned more or less on the center line of the heater. However, in correspondence of the frame 4, said wall is reduced to a thin strip 4b, so as to allow exposing the block 3 directly to the cavity defined by the frame 4. Such an arrangement allows to obtain a proper heating of the wick not only in correspondence of the wall side 4b, where it faces directly the block 3, but also in correspondence of the other wall sides 4a of the frame 4 which, due to their very low thickness, do not hinder the transfer of the constant heat flow which reaches their external surface as a result of the convective flows of the air heated by the block 3.

The frame 4 comprises moreover narrow tapered inner ribs 5 (one of them simply consisting of a projection in correspondence of the strip 4b), whose function is to contribute even further to the perfect centering of the wick into said frame 4, said effect being produced even if the wick were to find itself slightly out-of-axis in respect of the axis of the vessel into which it is housed. The ribs 5 are extremely narrow and, thanks to this and to the chimney effect produced inside the frame 4, they never reach exceedingly high temperatures; their contact with the wick thus never determines any phenomenon of carbonization of this latter, whereby said ribs are subject to no fouling.

According to the present invention, the heater comprises moreover, on the side opposite to the plug S, a housing 6 formed in one piece with and projecting from the support base 1, to hold a pilot light 7 - indicating the working condition of the electric heating element inserted into the block 3 - and the respective feeding circuit. A baffle 6a, inside the housing 6, provides to keep suitably parted the two bare wires feeding the light 7.

The heater of the present invention can finally be usefully equipped with a centering stem 8, which forms an engagement and rotation point for the rotary plug S, with its mushroom end 8a onto which said plug is snap engaged. It should be noted in this respect that - according to the teachings of the prior art - the plug S is already apt to rotate about a circular groove 12 of its own, cooperating with a corresponding rib of the outer shell containing the heater, so as to allow using said heater in a correct position both with horizontal and with vertical wall outlets. The presence of the pivoting joint 8a represents nonetheless, and first of all, a considerable advantage when mounting the heater, as it gives a precise positioning of the plug S which is extremely useful when carrying out the welding of the conductors 9 to said plug and when inserting the heater into its outer shell, making it also possible to automate these operations; said pivoting joint also helps to guarantee a further strength and lifetime of the rotary engagement between the plug S and the body of the evaporator.

All the aforedescribed elements of the heater according to the present invention are formed in one piece with the support base 1 during the molding process thereof, with no particular complications or additional costs, apart from the mold manufacturing costs. This contributes to notably simplify the construction of the two outer shells of the evaporator meant to house the heater, into which shells it will be sufficient to form the rib cooperating with the groove 12 of the plug S, as well as some ribs to keep in the wanted position the different parts of the heater and, in particular, the pilot light 7 with the respective feeding circuit and the conductors 9 feeding the electric heating element. The mounting of the heater thereby results extremely simplified and its functionality considerably improved.

There are no particular limitations as to the type of heating element adopted; one may thus indifferently use resistors, PTC elements with contact reeds, or PTC elements with metalized surfaces and welded terminals, provided that they are properly inserted and fixed into the block 3. In the first and last case, the electric heating elements will be usefully cemented into the ceramic block 3, whereas in the case of PTC elements with contact reeds, these latter will be suitably set into a recess of the ceramic block, using a system already known in technique.

## Claims

1. Heater for evaporators of insecticide or deodorant formulations both in solid mat form and in liquid form with a wick, of the type comprising at least one electric heating element, a flat surface for positioning the mat and a housing for the wick, characterized in that it comprises a support base (1) of plastic material consisting of a plate (1a), apt to form said flat surface, and of thin walls projecting from said plate, apt to define a frame (4) for housing said wick as well as a cage (2) for containing said electric heating element.

2. Heater as in claim 1), wherein said electric heating element is inserted and fixed into a ceramic block (3) preferably having the shape of a long parallelepipedon with square section.

3. Heater as in claim 2), wherein said plate comprises a window (1b) in correspondence of said cage (2), having dimensions slightly smaller than those of the side of said ceramic block (3) adjacent to said plate.

4. Heater as in claim 1), wherein the walls which define the frame for housing the wick are interrupted in correspondence of a zone in which said frame is adjacent to said cage.

5. Heater as in claim 1), wherein said frame for housing the wick comprises tapered inner ribs (5), parallel to the axis of the wick.

6. Heater as in claim 2), wherein said cage is delimited by said plate and by three walls projecting therefrom and apt to elastically cooperate with corresponding sides of said ceramic block.

7. Heater as in claim 6), wherein at least one of said walls comprises a terminal rib ending at right angles, in order to restrain said block.

8. Heater as in claim 1), wherein said support base comprises a housing formed in one piece therewith and projecting therefrom, to hold a pilot light (7) indicating the working condition of the electric heating element, and the respective feeding circuit.

9. Heater as in claim 1), wherein said support base comprises a stem (8), formed in one piece therewith and projecting therefrom, which constitutes an engagement and rotation point for an electric plug feeding the heater.

10. Heater as in claim 1) or 2), wherein said electric heating element is a resistor.

11. Heater as in claim 1) or 2), wherein said electric heating element is a PTC element with welded terminals.

12. Heater as in claim 10) or 11), wherein said electric heating elements are cemented into said ceramic block.

13. Heater as in claim 2), wherein said electric heating element is a PTC element with contact reeds inserted and fixed into a recess of the ceramic block.

## Patentansprüche

1. Heizelement für Verdampfer von Insektiziden oder Deodorantien in sowohl fester Mattenform als auch in flüssiger Form mit einem Docht vom Typ mit wenigstens einem elektrischen Heizelement, einer flachen Oberfläche zum Positionieren der Matte und einem Gehäuse für den Docht, dadurch gekennzeichnet, daß es eine Haltebasis (1) aus Kunststoff umfaßt, die aus einer Platte (1a), die gestaltet ist, um die flache Oberfläche zu bilden, und aus von der Platte vorragenden dünnen Wänden besteht, die gestaltet sind, um einen Rahmen (4) zum Unterbringen des Dochtes sowie einen Käfig (2) zum Aufnehmen des elektrischen Heizelements zu definieren.

2. Heizelement nach Anspruch 1, dadurch gekennzeichnet, daß das elektrische Heizelement in einen Keramikblock (3) eingesetzt und fixiert ist, der vorzugsweise die Gestalt eines langen Parallelpipedon mit quadratischem Querschnitt aufweist.

3. Heizelement nach Anspruch 2, dadurch gekennzeichnet, daß die Platte ein Fenster (1b) in Übereinstimmung mit dem Käfig (2) mit Abmessungen aufweist, die etwas geringer als diejenigen der Seite des Keramikblocks (3) benachbart zur Platte sind.

4. Heizelement nach Anspruch 1, dadurch gekennzeichnet, daß die Wände, die dem Rahmen zum Unterbringen des Dochtes definieren, in Übereinstimmung mit einem Gebiet unterbrochen sind, in dem der Rahmen sich benachbart zum Käfig befindet.

5. Heizelement nach Anspruch 1, dadurch gekennzeichnet, daß der Rahmen zum Unterbringen des Dochtes verjüngte innere Rippen (5) aufweist, die parallel zur Achse des Dochtes verlaufen.

6. Heizelement nach Anspruch 2, dadurch gekennzeichnet, daß der Käfig durch die Platte und durch drei davon vorragenden Wände begrenzt und gestaltet ist, um mit entsprechenden Seiten des Keramikblocks elastisch zusammenzuwirken.

7. Heizelement nach Anspruch 6, dadurch gekennzeichnet, daß wenigstens eine der Wände eine Abschlußrippe aufweist, die unter rechten Winkeln endet, um den Block festzuhalten.

8. Heizelement nach Anspruch 1, dadurch gekennzeichnet, daß die Haltebasis ein damit einteilig ausgebildetes und davon vorragendes Gehäuse, um ein Kontrollicht (7) zu halten, das den Arbeitszustand des elektrischen Heizelements anzeigt, und den jeweiligen Speiseschaltkreis aufweist.

9. Heizelement nach Anspruch 1, dadurch gekennzeichnet, daß die Haltebasis einen damit einteilig ausgebildeten und davon vorragenden Stift (8) aufweist, der einen Eingreif- und Drehpunkt für einen das Heizelement speisenden elektrischen Stecker bildet.

10. Heizelement nach Anspruch 1 oder 2, dadurch gekennzeichnet, daß das elektrische Heizelement ein Widerstand ist.

11. Heizelement nach Anspruch 1 oder 2, dadurch gekennzeichnet, daß das elektrische Heizelement ein PTC-Element mit angeschweißten Anschlüssen ist.

12. Heizelement nach Anspruch 10 oder 11, dadurch gekennzeichnet, daß die elektrischen Heizelemente in den Keramikblock zementiert sind.

13. Heizelement nach Anspruch 2, dadurch gekennzeichnet, daß das elektrische Heizelement ein PTC-Element mit in eine Aussparung des Keramikblocks eingesetzten und darin fixierten Kontaktzungen ist.

## Revendications

1. Réchauffeur d'évaporateurs de formulations d'insecticides ou de déodorants et sous forme de carte solide et sous forme liquide avec une mèche, du type comprenant au moins un élément chauffant électrique, une surface plate pour placer la carte et un boîtier pour la mèche, caractérisé en ce qu'il comprend une base (1) de support en matière plastique constituée d'une plaque (la), apte à former ladite surface plate, et de parois minces faisant saillie de ladite plaque, aptes à définir un bâti (4) destiné à loger ladite mèche, de même qu'une cage (2) destinée à contenir ledit élément chauffant électrique.

2. Réchauffeur selon la revendication 1, dans lequel ledit élément chauffant électrique est introduit et fixé dans un bloc en céramique (3) ayant, de préférence, la forme d'un parallélépipède long à section carrée.

3. Réchauffeur selon la revendication 2, dans lequel ladite plaque comprend une fenêtre (1b) en correspondance avec ladite cage (2), ayant des dimensions légèrement plus petites que celles du côté dudit bloc en céramique (3) adjacent à ladite plaque.

4. Réchauffeur selon la revendication 1, dans lequel les parois qui définissent le bâti destiné à loger la mèche sont interrompues en correspondance avec une zone dans laquelle ledit bâti est adjacent à ladite cage.

5. Réchauffeur selon la revendication 1, dans lequel ledit bâti destiné à loger la mèche comprend des nervures intérieures chanfreinées (5) parallèles à l'axe de la mèche.

6. Réchauffeur selon la revendication 2, dans lequel ladite cage est délimitée par ladite plaque et par trois parois faisant saillie de celle-ci et aptes à coopérer élastiquement avec les côtés correspondants dudit bloc en céramique.

7. Réchauffeur selon la revendication 6, dans lequel au moins une desdites parois comprend une nervure terminale se terminant à angles droits, afin de maintenir ledit bloc.

8. Réchauffeur selon la revendication 1, dans lequel ladite base de support comprend un boîtier formé d'un seul tenant avec et en saillie de celle-ci, pour contenir une veilleuse (7), indicative de l'état de marche de l'élément chauffant électrique, et le circuit d'alimentation correspondant.

9. Réchauffeur selon la revendication 1, dans lequel ladite base de support comprend une tige (8), formée d'un seul tenant avec et en saillie de celle-ci, laquelle constitue un point d'engagement et de rotation pour une prise de courant mâle alimentant le réchauffeur.

10. Réchauffeur selon la revendication 1 ou 2, dans lequel ledit élément chauffant électrique est une résistance.

11. Réchauffeur selon la revendication 1 ou 2, dans lequel ledit élément chauffant électrique est un élément PTC muni de bornes soudées.

12. Réchauffeur selon la revendication 10 ou 11, dans lequel lesdits éléments chauffants sont scellés dans ledit bloc en céramique.

13. Réchauffeur selon la revendication 2, dans lequel ledit élément chauffant électrique est un élément PTC muni de peignes de contact introduits et fixés dans un évidement du bloc en céramique.
